# EUROPÄISCHE PATENTSCHRIFT

(11) **EP 0 730 473 B1**
(45) Veröffentlichungstag und Bekanntmachung des Hinweises auf die Patenterteilung: **29.08.2001**
(21) Anmeldenummer: 95901432.5
(22) Anmeldetag: 25.11.1994
(51) Int. Cl.: A61K 47/48

(54) **HIRUDIN-KONJUGATE AUS HIRUDIN UND LIPOPHILEN VERBINDUNGEN**
HIRUDIN CONJUGATES FROM HIRUDIN AND LIPOPHILIC COMPOUNDS
CONJUGUES D'HIRUDINE COMPRENANT DE L'HIRUDINE ET DES COMPOSES LIPOPHILES

(30) Priorität: 02.12.1993 DE 4341115
(43) Veröffentlichungstag der Anmeldung: 11.09.1996
(73) Patentinhaber: BASF AKTIENGESELLSCHAFT, 67056 Ludwigshafen (DE)
(72) Erfinder: SCHWEDEN, Jürgen, D-67433 Neustadt (DE); ECKES, Peter, D-67166 Otterstadt (DE); HORNBERGER, Wilfried, D-67434 Neustadt (DE); SUBKOWSKI, Thomas, D-67112 Mutterstadt (DE)
(86) Internationale Anmeldenummer: EP9403901
(87) Internationale Veröffentlichungsnummer: WO9515183

(56) Entgegenhaltungen:
- EP-A- 0 077 529
- WO-A-89/07938
- WO-A-90/10448
- WO-A-91/08229
- WO-A-91/14696
- WO-A-92/19223
- WO-A-94/01138
- MOLECULAR IMMUNOLOGY, Bd. 21, no.1, 1984 Seiten 13-16, THOMAS P. HOPP 'IMMUNOGENICITY OF A SYNTHETIC HBsAg PEPTIDE: ENHANCEMENT BY CONJUGATION TO A FATTY ACID CARRIER.'

## Beschreibung

Die Erfindung betrifft neue Hirudin-Konjugate, die aus einem Hirudin und lipophilen Verbindungen gebildet werden, deren Herstellung sowie deren Verwendung als Arzneimittel.

Hirudin ist ein seit langer Zeit bekanntes, natürlich vorkommendes Protein mit blutgerinnungshemmenden Eigenschaften. Es ist der stärkste und selektivste bisher bekannte Thrombininhibitor (Naturwissenschaften, 42, 537, (1955); Hoppe-Seylers Z. für Biol. Chemie 366, 379 (1985)). Natürliches Hirudin ist eine Mischung von chemisch verwandten Peptiden mit einer Molmasse von 6900 - 7000 Dalton und 64 - 66 Aminosäuren. Bisher wurden etwa 20 natürlich vorkommende Hirudin-Varianten beschrieben (Scharf et al., FEBS-Letters 255, 105-110, (1989)).

In EP 345616 werden Kopplungsprodukte aus Hirudin und polymeren Trägern beschrieben. Als Träger werden lösliche und unlösliche Polymere wie Dextran, Sepharose, Heparin, Laevan und Gelatinepartialhydrolysat genannt. Die Träger-modifizierten Hirudine sollen verbesserte pharmakologische Eigenschaften wie etwa eine verlängerte Halbwertszeit besitzen.

In DE 40 14 260 werden Hirudinpolyalkylenglykolkonjugate beschrieben, die im Vergleich zum natürlichen Hirudin ein günstigeres pharmakologisches Wirkprofil, etwa eine verlängerte biologische Wirksamkeit und eine bessere Bioverfügbarkeit besitzen.

In den beiden obengenannten Offenlegungsschriften werden Hirudin-derivate mit verlängerter Wirksamkeit beschrieben, die durch Modifikation des Hirudins mit einem hochmolekularen Polymeren erreicht wird. Die Molmasse des Polymeren beträgt dabei einige Kilodalton, so daß die Molmasse des Polypeptids Hirudin erheblich vergrößert wird. Solche hochmolekularen Polymere sind jedoch chemisch nicht exakt definiert; sie bilden vielmehr eine Population verschiedener Moleküle, die sich um einen Molmassenbereich verteilt. Demzufolge bestehen auch die damit hergestellten Hirudin-Kopplungsprodukte nicht aus einer einzigen chemischen Verbindung, sondern aus Molekülpopulationen, die sich bezüglich ihrer Molmasse voneinander unterscheiden.

Es ist jedoch wünschenswert, Wirkstoffe, die als Arzneimittel eingesetzt werden, chemisch möglichst exakt zu definieren, um ihr Verhalten im Organismus besser kontrollieren zu können.

Es bestand daher die Aufgabe, Hirudinderivate mit gegenüber dem normalen Hirudin verbesserten pharmakologischen Eigenschaften bereitzustellen, die die obengenannten Nachteile der mit hochmolekularen Polymeren modifizierten Hirudine nicht besitzen.

Diese Aufgabe wird gelöst durch Hirudin-Konjugate, gebildet aus einem Hirudin und einem oder mehreren lipophilen Verbindungen, wobei die lipophile Verbindung einen Octanol-Wasser-Verteilungskoeffizienten von mehr als 1,8 aufweist und chemisch kovalent mit dem Hirudin verknüpft ist.

Für die erfindungsgemäßen Hirudin-Konjugate sind alle natürlich vorkommenden Hirudine und auch davon abgeleitete Varianten, sogenannte Hirudin-Muteine, und Bruchstücke mit thrombininhibierender Wirkung geeignet.

Solche Hirudine und ihre Herstellung sind beispielsweise aus EP 171 024, EP 158 986, DE 38 055 406, WO 92/1712, GB 2 247 239, EP 557 199, WO 92/5748, DE 40 14 260 bekannt.

Weiterhin sind von Hirudin strukturell abgeleitete Peptide oder Peptidderivate mit Hirudinaktivität, wie sie beispielsweise in EP 333 356 beschrieben werden, gut für die Herstellung der erfindungsgemäßen Hirudin-Konjugate geeignet.

Bevorzugt werden solche Hirudine verwendet, bei denen die Anknüpfung der lipophilen Verbindung zu keinem Aktivitätsverlust führt. Dies ist beispielsweise bei Hirudinen der Fall, bei denen die Anknüpfung über Seitenketten der Aminosäuren 27 bis 37 erfolgt. Besonders vorteilhaft für die Anknüpfung sind die Aminosäurepositionen 27 und 33 (bezogen auf das natürliche Hirudin HVI), da diese Verbindungen an der Spitze einer fingerartigen Region liegen und die Interaktion des Hirudins mit Thrombin nicht stören.

Die Bindung der lipophilen Verbindungen, gegebenenfalls des Spacers mit den lipophilen Verbindungen, kann an Aminogruppen des Hirudins, beispielsweise die freie N-terminale Aminogruppe, Aminogruppen der Lysinseitenketten, Aminogruppen der Histidine, Amidingruppen der Arginine, oder an Hydroxygruppen des Hirudins, beispielsweise von Tyrosin-, Serin- oder Threoninseitenketten erfolgen.

Besonders geeignet zur Anknüpfung der lipophilen Verbindungen sind die Aminogruppen der Lysinseitenketten.

Ebenfalls möglich ist die Bindung der lipophilen Verbindung an ein modifiziertes Tyrosin des Hirudins, das durch Nitrierung und Reduktion zugänglich ist (Meth. Enzymol. 25, 515-521, (1972)). An das gebildete Arylaminotyrosin kann dann die lipophile Verbindung mit bekannten Kopplungsmethoden angeknüpft werden.

Als lipophile Verbindung eignen sich Verbindungen, die über eine oder mehrere funktionelle Gruppen wie Amino-, Hydroxy-, Carboxy- oder Sulfonsäuregruppen verfügen und die einen Octanol-Wasser-Verteilungskoeffizienten von größer als 1,8 besitzen.

Die lipophilen Verbindungen können Naturstoffe sein, z.B. gesättigte oder ungesättigte Fettsäuren, Terpene, Prostaglandine, fettlösliche Vitamine, Carotinoide oder Steroide aber auch synthetische Carbonsäuren, Alkohole, Amine und Sulfonsäuren mit einem oder mehreren Alkyl-, Aryl-, Alkenyl oder auch mehrfach ungesättigten Verbindungen, die sowohl linear als auch verzweigt sein können und gegebenenfalls mit Halogen-, Nitro-, Cyano-, Alkoxy-, Alkylthio- oder Halogenalkylgruppen substituiert sind.

Beispiele für lipophile Verbindungen sind die gesättigten Fettsäuren Capronsäure, Caprylsäure, Caprinsäure, Laurinsäure, Myristinsäure, Palmitinsäure, Stearinsäure, Arachinsäure und Behensäure und die ungesättigten Fettsäuren Palmitoleinsäure, Ölsäure, Linolsäure, Linolensäure, Rizinolsäure, Octadecatetraensäure, Eicosaensäure, Eicosadiensäure, Arachidonsäure, Eicosapentaensäure oder Erucasäure sowie die aus ihnen erhältlichen Fettalkohole und Fettamine.

Geeignet sind auch Mischungen von Fettsäuren , wie man sie bei der Verseifung natürlicher Fette wie Kokosfett, Palmkernfett, Rapsöl, Olivenöl, Sonnenblumenöl, High Oleic Sonnenblumenöl, Ricinusöl oder Rindertalg erhält.

Weitere Beispiele für lipophile Verbindungen sind die Carotinoide Zeaxanthin, Rhodovibrin oder Astaxanthin, die Steroide Cholesterin Desmosterol, Coprosterol, Cerebrosterol, Lathosterol, Ergesterol, Sitosterol, Stigmasterol, Cholansäure, Cholinsäure, Dehydrocorticosterol, Aldosteron, Androsteron, Testosteron, Tachysterol, Lanosterol oder Lumisterol, die Terpene Geraniol, Nerol, Linalool, Menthol, Carveol, Borneol, Farnesol, Nerolidol oder Sclareol, die Prostaglandine Brefeldin, PGE₂ oder PGF₂, die Vitamine A₁ oder D aber auch synthetische Verbindungen wie Oxoalkohole, Hexylamin, Ethylhexansäure, Ethylhexanol, Ethylhexylamin oder Alkylbenzolsulfonsäuren.

Für die Erfindung besonders geeignete lipophile Verbindungen sind solche der allgemeinen Formel I 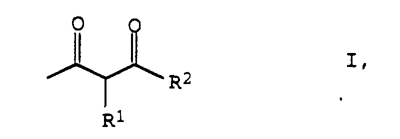 worin
- R¹ und R²: unabhängig voneinander
(CH₂)ₘ-C(R³)(R⁴)-(CH₂)ₙ-(CH=CH-CH₂)ₒ-CH₃,
- m: die Zahl 0 bis 28,
- n: die Zahl 3 bis 6,
- o: die Zahl 0 bis 6,
- R³ und R⁴: unabhängig voneinander H, C₁₋₆-Alkyl, C₃₋₆-Cycloalkyl, Aryl oder Benzyl, gegebenenfalls substituiert durch Halogen-, Nitro-, Cyano-, Alkyl-, Alkoxy-, Alkylthio-, Halogenalkylgruppen,
bedeuten.

Besonders bevorzugte Verbindungen der Formel I sind solche, bei denen die Reste R¹ und R² aus 4 bis 16 C-Atomen bestehen und die gesättigt oder ein- zwei- oder dreifach ungesättigt sind.

Als lipophile Verbindungen eignen sich weiter solche der allgemeinen Formel II

H-Y-R¹ II

worin
- R¹: (CH₂)ₘ-C(R³)(R⁴)-(CH₂)ₙ-(CH=CH-CH₂)ₒ-CH₃ und
- Y: -C(O)-, -N(R⁵)-, -O- mit
- R⁵: H, C₁₋₁₈-Alkyl, C₃₋₁₈-Alkenyl, C₃₋₆-Cycloalkyl, Aryl oder Benzyl, gegebenenfalls substituiert durch Halogen-, Nitro-, Cyano-, Alkyl-, Alkoxy-, Alkylthio-, Halogenalkylgruppen,
bedeuten.

Die Herstellung der erfindungsgemäßen Hirudin-Konjugate erfolgt indem ein Hirudin entweder direkt oder mittels eines Spacers mit einer oder mehreren lipophilen Verbindungen verknüpft wird.

Als Spacer sind alle bi- oder multifunktionellen Moleküle geeignet, die aufgrund ihrer multifunktionellen Gruppen eine Verknüpfung - gegebenenfalls nach Aktivierung - von Hirudin und der lipophilen Verbindung erlauben.

Als Spacer eignen sich besonders Aminosäuren, Oligopeptide, Mono-, Di- oder Oligosaccharide, Amino- oder Hydroxycarbonsauren, insbesondere solche mit einer Kettenlänge von 2 bis 10 C-Atomen, die gegebenenfalls zur Erhöhung der Hydrophilie weitere Substituenten tragen können, beispielsweise C₁-C₄-Oligoalkylenglykole.

Für die Anknüpfung von lipophilen Verbindungen an Hirudin können beispielsweise folgende Spacer eingesetzt werden:

―X―CO―,

―X―CO―NH―Z―NH―CO―,

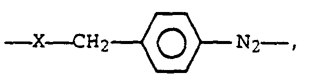

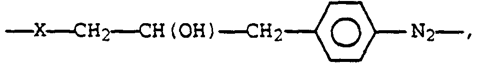

―X―CO―CH₂―CH₂―CO―,

―X―CH₂―CO―,

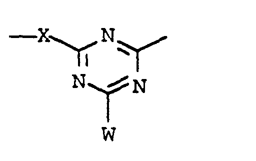

wobei
X für S, O, NH, N(CH₃), N(C₂H₅)
W für H, OH, Cl
Z für eine C₂-C₆-Alkylengruppe oder eine p-Phenylengruppe steht.

Für den Erhalt der biologischen Aktivität der Hirudin-Konjugate nach Bindung an Oberflächen wie Zellmembranen oder synthetischen Oberflächen kann die Verwendung eines Abstand vermittelnden Spacers erforderlich sein.

Für die erfindungsgemäßen Hirudin-Konjugate sind alle diejenigen lipophilen Verbindungen geeignet, die eine oder mehrere funktionelle Gruppen, wie beispielsweise Amino-, Hydroxy-, Carboxy- oder Sulfonsäuregruppen tragen, da diese funktionellen Gruppen nach Aktivierung mit den reaktiven Gruppen des Hirudins reagieren können.

Die Art der Aktivierung der lipophilen Verbindungen, gegebenenfalls die Verknüpfung über einen Spacer, hängt von der funktionellen Gruppe ab und kann in dem Fachmann bekannter Weise vorgenommen werden.

Trägt die lipophile Verbindung oder der Spacer mit dem lipophilen Verbindung eine Carboxylgruppe, so kann die Aktivierung beispielsweise durch Überführung in einen Alkenylester, Arylester, ein O-Halbacetal, O-Acylhalbaminoacetal, O-Acylhalbketal, O-Acylhalbaminoketal, O-Acyllactim, symmetrisches oder unsymmmetrisches Carbonsäureanhydrid, Carbonsäure-Carbaminsäureanhydrid, O-Acylisoureid, O-Acyl-N-alkylhydroxylamin, eine O-Acylhydroxamsäure, ein O-Acyl-N,N-diacylhydroxylamin, O-Acyloxim, O-Acyl-N-azo-N-acylhydroxylamin, O-Acyl-N-azo-N-arylhydroxylamin, Carbonsäure-Schwefligsäureanhydrid, Carbonsäure-Schwefelsäureanhydrid, Carbonsäure-Phosphorigsäureanhydrid, Carbonsäure-Phosphorsäureanhydrid, eine Acyloxyphosphoniumverbindung, ein Carbonsäure-Phosphorsäureamid-Anhydrid, Carbonsäurefluorid, oder Carbonsäurechlorid erfolgen, wie es in Müller, Houben-Weyl, "Methoden der organischen Chemie", Vol. 15/1 und 15/2 ,Thieme Verlag, Stuttgart (1974) oder Bodanszky, Klausner, Ondetti, "Peptide Synthesis", Seite 85-128, John Wiley & Sons, New York, 1976 oder anderen Standardwerken der Peptidchemie beschrieben ist.

Besonders geeignete reaktive Gruppen sind Carbonsäurechloride, symmetrische Anhydride, gegebenenfalls durch 1, 2, 3 oder 5 Halogen- oder Nitrogruppen substituierte Arylester, N-Hydroxysuccinimidester, N-Hydroxyphthalimidester oder N-hydroxybenzotriazolester.

In analoger Weise lassen sich Sulfonsäuregruppen aktivieren.

Liegen als funktionelle Gruppen in den lipophilen Verbindungen Amino- oder Hydroxygruppen vor, so erfolgt die Anknüpfung der lipophilen Verbindungen an das Hirudin bevorzugt über einen Spacer, der als zweite funktionelle Gruppe eine Carboxyl- oder Sulfonsäuregruppe trägt und in der oben beschriebenen Weise für die Kopplung an das Hirudin aktiviert wird.

Gut geeignet als lipophile Verbindungen sind auch Alkyldiketene, die ohne weitere Aktivierung direkt mit dem Hirudin verknüpft werden können.

Als Alkyldiketene bevorzugt verwendet werden Verbindungen der allgemeinen Formel III 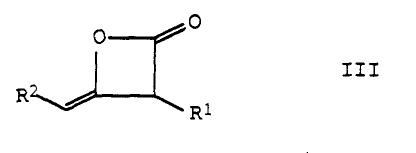 worin
- R¹ und R²: unabhängig voneinander
(CH₂)ₘ-C(R³) (R⁴)-(CH₂)ₙ-(CH=CH-CH₂)ₒ-CH₃,
- m: die Zahl 0 bis 28,
- n: die Zahl 3 bis 6,
- o: die Zahl 0 bis 6 und
- R³ und R⁴: unabhängig voneinander H, C₁₋₆-Alkyl, C₃₋₆-Cycloalkyl, Aryl oder Benzyl, gegebenenfalls substituiert durch Halogen-, Nitro-, Cyano-, Alkyl-, Alkoxy-, Alkylthio-, Halogenalkylgruppen,
bedeuten.

Besonders bevorzugte Verbindungen der allgemeinen Formel III sind Fettalkyldiketene, die sich wie beispielsweise in DE 2927118 beschrieben, von den gesättigten Fettsäuren Capronsäure, Caprylsäure, Caprinsäure, Laurinsäure, Myristinsäure, Palmitinsäure, Stearinsäure, Arachinsäure und Behensäure und den ungesättigten Fettsäuren Palmitoleinsäure, Ölsäure, Linolsäure, Linolensäure, Rizinolsäure, Octadecatetraensäure, Eicosaensäure, Eicosadiensäure, Arachidonsäure, Eicosapentaensäure oder Erucasäure ableiten.

Ferner können auch Fettalkyldiketene verwendet werden, die sich aus Mischungen von Fettsäuren ableiten, wie man sie bei der Verseifung natürlicher Fette wie Kokosfett, Palmkernfett, Rapsöl, Olivenöl, Sonnenblumenöl, High Oleic Sonnenblumenöl, Ricinusöl oder Rindertalg erhält.

Die erfindungsgemäß beschriebenen Hirudin-Konjugate sind chemisch exakt definiert und zeigen im Vergleich zu Hirudin ein günstigeres pharmakologisches Wirkprofil. Sie besitzen unter anderem eine erheblich verlängerte biologische Wirksamkeit und eine bessere Bioverfügbarkeit. Darüber hinaus erlauben die hydrophoben Hirudine ein Targeting des Wirkstoffs auf die Oberflächen von Blutzellen und Blutgefäßoberflächen.

Aufgrund dieser Eigenschaften sind die erfindungsgemäßen Hirudin-Konjugate wertvolle Arzneimittel zur Behandlung und Prophylaxe von thrombinabhängigen thromboembolischen Ereignissen wie tiefen Venenthrombose, Lungenembolien, Myocard- oder Cerebralinfarkten und instabiler Angina, weiterhin zur Therapie der Disseminierten Intravasalen Koagulation (DIC) sowie als Komedikation zu Thrombolytika wie Streptokinase, Urokinase, Prourokinase, t-PA, APSAC, Plasminogenaktivatoren aus den Speicheldrüsen von Tieren, sowie die rekombinanten und mutierten Formen all dieser Substanzen zur Verkürzung der Reperfusionszeit und Verlängerung der Reokklusionszeit.

Ein weiteres Anwendungsgebiet ist die Verhinderung thrombinabhängiger früher Reokklusion und später Restenosierung nach PTCA, die Verhinderung thrombininduzierter Proliferation glatter Muskelzellen, die Verhinderung der Akkumulation aktiven Thrombins im ZNS (z.B. bei M. Alzheimer), die Tumorbekämpfung und die Verhinderung von Mechanismen, die zu Adhäsion und Metastasierung von Tumorzellen führen.

Die neuen Verbindungen können in den gebräuchlichen galenischen Applikationsformen fest oder flüssig angewendet werden, z.B. als Lösungen, Salben, Cremes oder Sprays. Diese werden in üblicher Weise hergestellt. Die Wirkstoffe können dabei mit den üblichen galenischen Hilfsmitteln wie Füllstoffen, Konservierungsmitteln, Fließreguliermitteln, Netzmitteln, Dispergiermitteln, Emulgatoren, Lösungsmitteln und/oder Treibgasen verarbeitet werden (vgl. H. Sucker et al: Pharmazeutische Technologie, Thieme-Verlag, Stuttgart, 1978).

Die hydrophoben Eigenschaften ermöglichen auch eine transdermale Applikation der neuen Hirudinderivate besonders in Verbindung mit Penetrationsverstärkern wie z.B. Dimethylsulfoxid, Alkoholen oder auch Azonen bzw. durch Iontophorese.

Die Dosierung hängt vom Alter, Zustand und Gewicht des Patienten sowie von der Applikationsart ab. Je nach Applikationsform und Indikation beträgt die tägliche Wirkstoffdosis in der Regel zwischen etwa 20 bis 40.000 ATU/kg Körpergewicht.

Die Hirudin-Konjugate können auch erfolgreich zur antithrombogenen Beschichtung von künstlichen Oberflächen, wie beispielsweise Hämodialysemembranen und den dazu erforderlichen Schlauchsystemen, bei Gefaßersatz oder bei Herz-Lungenmaschinen eingesetzt werden.

Die folgenden Beispiele dienen der weiteren Beschreibung der Erfindung.

### Beispiel 1

### Octyldiketen

400,1 g N,N-Dimethylcyclohexylamin wurden in 2 1 Toluol bei 50 bis 60°C gelöst und bei dieser Temperatur 487,5 g Octansäurechlorid in 2,5 Std. zugetropft. Es wurde zwei Stunden bei 50 bis 60°C nachgerührt und auf Raumtemperatur gekühlt.

Die Reaktionsmischung wurde mit eiskalter 1 N Schwefelsäure, Wasser und gesättigter Natriumchloridlösung gewaschen und die organische Phase über Magnesiumsulfat getrocknet. Das Rohprodukt wurde durch fraktionierende Destillation gereinigt. Als Hauptfraktion wurden 317,3 g einer farblosen Flüssigkeit bei einer Kopftemperatur von 135°C und einem Druck von 0,3 mbar erhalten. Das Produkt besteht nach GC-Analyse zu 97 % aus Octyldiketen. IR-, ¹H- und ¹³C-NMR-Spektra stimmen mit der vorgeschlagenen Struktur überein.

### Beispiel 2

### Kopplung von Octyl-Diketen an Hirudin

Als Hirudin wurde ein Hirudinmutein verwendet, das sich vom natürlichen Hirudin HV1 durch folgende Aminosäureaustausche unterscheidet: Pos. 27:Lys; Pos. 33:Lys; Pos. 36:Arg; Pos. 47:Arg. Die Herstellung eines solchen Hirudinmuteins ist in DE 40 14 260 beschrieben.

20 mg Hirudinmutein wurden in 1 ml 100 mM Natriumboratpuffer pH 9,0 gelöst und mit 1 ml n-Propanol und 1,5 mg Octyldiketen versetzt und anschließend unter intensivem Rühren bei 4°C inkubiert.

Nach 30-minütiger Reaktionszeit wurde das nicht umgesetzte Octyl-diketen durch Extraktion mit n-Hexan (1 Vol/Vol) abgetrennt. Die entstandene Produktmischung wurde nach 50-fachem Verdünnen mit 0,1 % TFA/H₂O auf eine Hi-Pore RP 304 Säule (250 x 4,6 cm, Biorad, Nr. 125-0550) aufgetragen und mit folgendem Gradienten entwickelt:
- Lösung A:: 0,1 % TFA/H₂O
- Lösung B:: 0,1 % TFA/Acetonitril
- Flußrate:: 1 ml/min
- Detektion:: 216 nm

| Zeit (min) | Anteil B (%) |
|---|---|
| 0 | 0 |
| 40 | 50 |
| 40,5 | 100 |
| 45 | 100 |
| 45,5 | 0 |

Die Hirudin-Diketen-Komplexe eluieren unter folgenden Bedingungen:

| | % B |
|---|---|
| 1 Diketen-Einheit pro Hirudin (Octyl-Diketen₁-Hirudin) | 56 % |
| 2 Diketen-Einheiten pro Hirudin (Octyl-Diketen₂-Hirudin) | 60 % |
| 3 Diketen-Einheiten pro Hirudin (Octyl-Diketen₃-Hirudin) | 68 % |

Die spezifische Aktivität der aus dem Kopplungsansatz isolierten Derivate betrug für

| | ATU/mg |
|---|---|
| Octyl-Diketen₁-Hirudin | 7000 |
| Octyl-Diketen₂-Hirudin | 8000 |
| Octyl-Diketen₃-Hirudin | 4900 |

(bestimmt durch Thrombininhibitionstest mit dem chromogenen Substrat S 2238 (Kabi-Pharmacia), FEBS-Lett. (1983) 164, 307, Proteinbestimmung mittels UV-Extinktion (εₘₒₗₐᵣ = 3148, ¹/_{Mol.cm}; λ = 278 nm)

### Beispiel 3

### Hexyldiketen

272,2 g N,N-Dimethylcyclohexylamin wurden in 1 l Toluol vorgelegt und für 2 Std. am Wasserabscheider Wasser ausgekreist. Man ließ auf 50 bis 60°C abkühlen, tropfte 269 g Hexansäurechlorid zu und rührte zwei Stunden bei 55°C nach.

Die Reaktionsmischung wurde mit 1 N Schwefelsäure, Wasser und gesättigter Kochsalzlösung gewaschen und die organische Phase über Magnesiumsulfat getrocknet. Das Rohprodukt wurde durch fraktionierende Destillation gereinigt. Als Hauptfraktion werden 172,8 g einer farblosen Flüssigkeit bei einer Übergangstemperatur von 95 bis 100°C und einem Druck von 0,15 mbar erhalten. Das Produkt besteht nach GC-Analyse zu 98 % aus Hexyldiketen. IR-, ¹H- und ¹³C-NMR-Spektra stimmen mit der vorgeschlagenen Struktur überein.

### Beispiel 4

### Kopplung von Hexyl-Diketen an Hirudin

Die Kopplung von Hexyl-Diketen an Hirudin erfolgte analog zu Beispiel 2. Für die Reaktion werden 20 mg Hirudinmutein gelöst in 2 ml 50 % n-Propanol, 50 mM Na-Carbonat pH 9,5 eingesetzt. Die Trennung der Produktmischung erfolgt nach Abstoppen (siehe Bsp. 2) auf einer RP 304 HPLC-Säule (Biorad Nr. 125-0550) mit dem folgende Gradienten:
- Lösungsmittel A:: 5 mM Ammoniumacetat pH 6,5
- Lösungsmittel B:: Methanol
- Flußrate:: 1 ml/min
- Detektion:: 216 nm

| Zeit (min) | Anteil B (%) |
|---|---|
| 0 | 0 |
| 5 | 30 |
| 30 | 70 |
| 30,1 | 100 |
| 35,0 | 100 |
| 35,1 | 0 |

Die Kopplungsprodukte Hexyl-Diketen₁-Hirudin, Hexyl-Diketen₂-Hirudin u. Hexyl-Diketen₃-Hirudin eluieren im Gradienten bei 42,5, 44 und 50 % Lösungsmittel B. Die spezifische Aktivität wurde wie im Bsp. 2 beschrieben bestimmt und betrug für das Hexyl-Diketen₃-Hirudin 5700 ATU/mg.

### Beispiel 5

### Sonnenblumenfettsäurechlorid

70,5 g Sonnenblumenfettsäure aus High Oleic Sunflower Oil mit einem Ölsäuregehalt von 89 % und der Säurezahl 219 wurden in 250 ml Toluol gelöst und auf 40°C erwärmt. Bei dieser Temperatur wurden 41,2 g Oxalylchlorid zugetropft und zwei Stunden nachgerührt.

Das Lösungsmittel wurde bei 60°C und reduziertem Druck destillativ entfernt. Man erhielt 75 g Sonnenblumenfettsäurechlorid als gelbes Öl mit der Chloridzahl 12,0.

### Beispiel 6

### Alkyldiketen aus Sonnenblumenfettsäurechlorid (Sunnyldiketen)

65 g Sonnenblumenfettsäurechlorid der Chloridzahl 12,0 wurden in 400 ml Toluol vorgelegt und auf 50°C erwärmt. Bei dieser Temperatur tropfte man in 25 min. 33,7 g N,N-Dimethylcyclohexylamin zu. Die Reaktionsmischung wurde nach zwei Stunden bei 50°C gehalten und dann auf Raumtemperatur abgekühlt.

Der Feststoff wurde durch Druckfiltration abgetrennt und die organische Phase mit 1 M Schwefelsäure, gesättigter Natriumhydrogencarbonat- und gesättigter Natriumchloridlösung gewaschen. Nach Trocknen der organischen Phase über Magnesiumsulfat wurde das Lösungsmittel bei reduziertem Druck destillativ entfernt. Es verblieben 51,8 g einer schwach gelben öligen Flüssigkeit, die laut ¹H- und ¹³C-NMR zu 95 % aus dem Alkyldiketen bestand.

### Beispiel 7

### Herstellung von Sunnyldiketen-Hirudin

20 mg Hirudinmutein (wie in Beispiel 2) wurden in 2 ml 50 % n-Propanol, 50 mM Kaliumcarbonatpuffer pH 9,5 gelöst mit 8,5 mg Sunnyldiketen versetzt und 60 min. bei 20°C unter intensivem Rühren inkubiert. Das überschüssige Diketen wurde nach Ablauf der Reaktionszeit mit n-Hexan (1 Vol/Vol) extrahiert. Die Produktmischung wurde zur Trennung auf eine RP-304 HPLC-Säule (Bio-Rad. Nr. 125-0550; 250 x 4,6 cm) aufgetragen und die Säule dann mit folgendem Gradienten eluiert:
- Lösungsmittel A:: 5 mM Ammoniumacetat pH 6,5
- Lösungsmittel B:: Methanol
- Flußrate:: 1 ml/min
- Detektion:: 216 nm

| Zeit (min) | Anteil B (%) |
|---|---|
| 0 | 0 |
| 5 | 70 |
| 30 | 95 |
| 30,1 | 100 |
| 35,0 | 100 |
| 35,1 | 0 |

Die Sunnyldiketen-Hirudin-Konjugate Sunnyl₁-Hirudin, Sunnyl₂-Hirudin und Sunnyl₃-Hirudin eluieren bei 77 %, 80 % und 90 % Methanol.

Die spezifischen Aktivitäten wurden wie in Bsp. 2 ermittelt und betrugen
7700 ATU/mg für Sunnyl₁-Hirudin
7100 ATU/mg für Sunnyl₂-Hirudin
6100 ATU/mg für Sunnyl₃-Hirudin

### Beispiel 8

### Umsetzung von Palmitinsäure-N-Hydroxysuccinimidester mit Hirudin

20 mg Hirudinmutein (wie in Beispiel 2) wurden in 1 ml 100 mM Natriumcarbonat pH 9,5 gelöst und mit 1 ml einer Lösung von 1,5 mg Palmitinsaure-N-Hydroxy-succinimidester (Sigma) in 1,4-Dioxan versetzt und bei 20°C unter Rühren zur Reaktion gebracht. Nach einer Reaktionszeit von 4 h wurde durch Zusatz von einem 5fach molaren Überschuß von Butylamin die Reaktion mit dem Hirudin abgestoppt (eine Stunde bei Raumtemperatur).

Die Auftrennung der Produktmischung erfolgte auf einer RP-304 HPLC-Säule (Biorad 125-0550) im folgendem Gradienten:
- Lösungsmittel A:: 5 mM Ammoniumacetat pH 6,5
- Lösungsmittel B:: 100 % Methanol
- Flußrate:: 1 ml/min
- Detektion:: 216 nm

| Zeit (min) | Anteil B (%) |
|---|---|
| 0 | 0 |
| 5 | 40 |
| 30 | 90 |
| 30,1 | 100 |
| 35 | 100 |
| 35,1 | 0 |

Das Palmitinsäure₁-Hirudin eluiert bei 57 %, das Palmitinsäure₂-Hirudin bei 62 % und das Palmitinsäure₃-Hirudin bei 76 % Lösungsmittel B.

### Beispiel 9

### Herstellung von Ölsäure-Hirudin

2 g Ölsäureanhydrid wurde in 4 ml Dioxan gelöst, mit 460 mg N-Hydroxysuccinimid versetzt und 2 h bei Raumtemperatur gerührt. 200 µl dieser Lösung der aktivierten Ölsäure wurden dann mit 20 ml n-Propanol und 20 ml einer Hirudinlösung (20 mg/ml) in 0,1 M Na-Carbonat oder Na-Borat pH 9 versetzt und 4 h bei Raumtemperatur inkubiert. Die Reaktion wurde durch Zusatz von Ethanolamin (2fach molarer Überschuß bezogen auf Ölsäure) abgestoppt.

2 ml des Kopplungsansatzes wurden mit 40 ml 20 mM Na-Phosphatpuffer pH 7,0 3,5 M NaCl (Laufmittel A) verdünnt und auf eine TSK-Butyl-Säule (1 cm x 28 cm, Vol: 22 ml, Beladung 2 mg/ml Gel) aufgetragen und die Säule bei einem Fluß von 2 ml/min mit 6 Säulenvolumen (SV) mit Laufmittel A gewaschen und mit folgenden Gradienten (Laufmittel B: 20 mM Na-Phosphat pH 7,0) entwickelt:
1. 2,5 SV 70 % B
2. 2,5 SV 80 % B
3. 2,5 SV 90 % B
4. 2,5 SV 100 % B
5. 2,5 SV 30 % Methanol in B
6. 2,5 SV 40 % Methanol in B

Die Hirudin-Derivate eluierten bei 100 % B (Ölsäure₁-Hirudin und Ölsäure₂-Hirudin) und 30 % Methanol (Ölsäure₃-Hirudin). Die spezifischen Aktivitäten betrugen 11000 U/mg für Ölsäure₁-Hirudin, 6200 U/mg für Ölsäure₂-Hirudin und 6600 U/mg für Ölsäure₃-Hirudin.

### Beispiel 10

### Herstellung von Cholesterin-Hirudin

1 ml einer Hirudin-Lösung (20 mg/ml in 0,1 M Na-Carbonat oder Na-Borat pH 9,5) wurden mit einer Lösung von mit 3,2 mg N-Hydroxysuccinimid aktiviertem Cholesterin in 1,2 ml THF versetzt und 4 h bei Raumtemperatur inkubiert. Die Reaktion wurde durch Zusatz von Ethanolamin abgestoppt (2fach molarer Überschuß bezogen auf Cholesterin) und der pH auf 7,0 eingestellt.

2 ml des Kopplungsansatzes wurden mit 8 ml Ammoniumacetat-Lösung (2 mM, pH 6,0, Laufmittel A) verdünnt und bei einer Flußrate von 2,5 ml/min auf eine HiPore BioRad RP 304-Säule (10 x 250 mm) aufgetragen und mit folgendem Gradienten entwickelt:

| Minuten | % B |
|---|---|
| 0 | 0 |
| 10 | 40 |
| 60 | 90 |

Das Cholesterin-Hirudin eluierte bei 38 Minuten. Die spezifische Aktivität des Derivates betrug 16000 U/mg.

Das aktivierte Cholesterin wurde wie folgt hergestellt:

15,0 g 2β-Cholesterol und 5,2 g Bernsteinsäureanhydrid wurden in 40 ml Pyridin 22 h zum Rückfluß erhitzt und danach in 250 ml MTBE aufgenommen. Die organische Phase wurde mit 200 ml 1N Salzsäure, 200 ml Wasser und 200 ml gesättigter Natriumchloridlösung extrahiert und mit Magnesiumsulfat und Aktivkohle aufgerührt. Das Filtrat wurde im Vakuum vom Lösungsmittel befreit. Es resultierten 18,1 g Bernsteinsäure-cholest-5-en-3β-yl-ester.

1,94 g des so erhaltenen Esters und 0,55 g N-Hydroxysuccinimid wurden in 12 ml Dichlormethan bei 4°C vorgelegt und 1,01 g N,N-Dicyclohexylcarbodiimid zugefügt. Man ließ auf Raumtemperatur erwärmen, rührte über Nacht und filtrierte den Feststoff ab. Das in der organischen Phase enthaltene Rohprodukt wurde an 80 g Kieselgel chomatographiert (Dichlormethan + Essigsäureethylester=2+1). Man erhielt 1,7 g mit N-Hydroxysuccinimid aktiviertes Cholesterin (N-[3-(Cholest-5-en-3β-yloxycarbonyl)-propionyloxy]succinimid).

### Beispiel 11

### Herstellung von Farnesyl-Hirudin

5,6 ml einer Hirudin-Lösung (21 mg/ml in 0,1 M Na-Carbonat pH 9) wurden mit 5,5 ml n-Propanol verdünnt und mit 32 mg Farnesylalkohol-p-nitrophenylcarbonat versetzt und 12 h bei Raumtemperatur unter Rühren inkubiert. Der Reaktionsansatz wurde dann mit 150 ml 2 M NaCl, 20 mM Na-Phosphat pH 7 verdünnt und auf eine t-Butyl-Säule aufgetragen (Beladung 2 mg Protein/ml Gel, Säulenhöhe 25 cm). Anschließend wurde die Säule mit 2,2 Säulenvolumen Auftragspuffer gewaschen und dann mit einem linearen Gradienten von 2 M NaCl, 20 mM Na-Phosphat pH 7 nach 20 mM Na-Phosphat pH 7 (25 Säulenvolumen) entwickelt. Das Farnesyl-Hirudin eluierte am Ende des Gradienten bei 20 mM Na-Phosphat. Die spezifische Aktivität wurde wie in Beispiel 2 ermittelt und betrug 11300 U/mg.

Das Farnesylalkohol-p-nitrophenylcarbonat wurde wie folgt hergestellt:

1,40 g Farnesol und 1,01 g Diazo-[2,2,2]-bicyclooctan wurden in 20 ml Dichlormethan gelöst und in 15 Minuten 3,63 g p-Nitrophenylchlorformiat in 10 ml Dichlormethan zugetropft. Man rührte 1,5 Std., entfernte das Lösungsmittel im Vakuum und chromatographierte das Rohprodukt an 150 g Kieselgel (Hexan + Essigsäureethylester = 20+1). Man erhielt 2,2 g Farnesylalkohol-p-nitrophenylcarbonat.

### Beispiel 12

### Herstellung von Stearinsäure-Hirudin

0,5 g Stearoylchlorid wurden in 2 ml 1,4 Dioxan gelöst und mit 0,29 g N-Hydroxysuccinimid sowie 0,1 ml Triethylamin versetzt und 3 h bei Raumtemperatur gerührt. Die Reaktionsmischung wurde mit 1 ml Wasser versetzt und der pH durch Laugenzugabe auf 5 eingestellt. 60 µl der succinimidaktivierten Stearinsäure-Lösung wurden mit 2,5 ml Hirudin-Lösung (21 mg/ml in 0,1 M Na-Carbonat pH 9) und 2,5 ml Dioxan vermischt und 12 h bei Raumtemperatur unter Rühren inkubiert. Der Reaktionsansatz wurde anschließend durch präparative RP-HPLC (siehe Beispiel 2) gereinigt. Die Derivate eluieren bei 48 % B, 54 % B, 62 % B und 74 % B. Die spezifischen Aktivitäten betrugen 9600 U/mg, 12400 U/mg, 12800 U/mg sowie 840 U/mg.

### Beispiel 13

### Herstellung von Lutensol TO3-Hirudin

0,5 ml einer Hirudin-Lösung (21 mg/ml in 0,1 M Na-Carbonat oder Na-Borat pH 9) wurden mit 0,5 ml Tetrahydrofuran verdünnt und mit 1,2 mg Lutensol TO3-N-Hydroxysuccinimidcarbonat (Beispiel 12) versetzt und 12 h bei Raumtemperatur unter Rühren inkubiert. Die Reaktion wurde durch Zusatz von Ethanolamin (2fach molarer Überschuß bezogen auf aktiviertes Lutensol) abgestoppt und durch Chromatographie - wie in Beispiel 10 beschrieben - aufgetrennt. Die Hirudin-Lutensol Derivate eluierten bei 45 % B (Derivat 1 11300 U/mg, für Derivat 2 9700 U/mg und 440 U/mg für Derivat 3.

Das Ausgangsmaterial wurde wie folgt hergestellt:

20 g Phosgen wurden bei 0°C in 100 ml Toluol einkondensiert und 34,0 g Lutensol T03 (OH-Zahl = 165) in 50 ml Toluol in 10 min zugetropft. Man ließ auf Raumtemperatur erwärmen und entfernte das Lösungsmittel bei 35°C im Ölpumpenvakuum. Es wurden 40,3 g des Chlorcarbonats von Lutensol TO3 erhalten.

1,2 g N-Hydroxysuccinimid und 1,0 g Triethylamin wurden in 20 ml Dichlormethan gelöst und 3,92 g Chlorcarbonat von Lutensol TO3 in 10 ml Dichlormethan in 5 min zugetropft: Man ließ 2 h bei Raumtemperatur nachrühren, entfernte das Lösungsmittel im Vakuum und nahm den verbleibenden Rückstand in Essigsäureethylester auf. Der verbleibende Feststoff wurde abgetrennt und das Filtrat im Vakuum von Lösungsmittel befreit. Man erhielt 4,3 g des N-Hydroxysuccinimidcarbonats von Lutensol TO3.

### Beispiel 14

### Herstellung von Octyl-Diketen-Phenylalanin-Hirudin

0,5 ml einer Hirudin-Lösung (21 mg/ml in 0,1 M Na-Carbonat oder Na-Borat pH 9) wurden mit 0,5 ml Tetrahydrofuran verdünnt und mit 4 mg N-(2-Hexyl-3-oxo-decanoyl)-phenylalanin-N-hydroxysuccinimidester versetzt und 12 h bei Raumtemperatur unter Rühren inkubiert. Die Reaktion wurde durch Zusatz von Ethanolamin (2fach molarer Überschuß bezogen auf aktiviertes Phenylalanin) abgestoppt und durch RP-HPLC - wie in Beispiel 10 beschrieben - aufgetrennt. Die Hirudin-Diketenderivate eluierten bei 43 % B (Derivat 1), 48 % B (Derivat 2) und 58 % B (Derivat 3). Die spezifischen Aktivitäten wurden wie in Beispiel 2 ermittelt und betrugen für Derivat 1 930 U/mg, für Derivat 2 2800 U/mg und 135 U/mg für Derivat 3.

Das Ausgangsmaterial wurde wie folgt hergestellt:

82,5 g Phenylalanin wurden in 500 ml Wasser bei pH 13,5 vorgelegt und in 1,25 h 145,9 g Octyldiketen in 50 ml Dichlormethan zugetropft. Die Suspension wurde 5 h intensiv gerührt, mit 2,5 l Wasser versetzt und mit konzentrierter Salzsäure auf pH 1 eingestellt. Die Wasserphase wurde mit insgesamt 2,5 l Dichlormethan extrahiert, die gesammelten organischen Phasen über Magnesiumsulfat getrocknet und das Lösungsmittel im Vakuum entfernt. Der resultierende weiße Feststoff wurde in 1000 ml Pentan aufgerührt, der Feststoff abfiltriert und bei 60°C im Vakuum getrocknet. Es resultierten 190 g N-(2-Hexyl-3-oxo-decanoyl)-phenylalanin.

N-(2-Hexyl-3-oxo-decanoyl)-phenylalanin und 0,55 g N-Hydroxysuccinimid wurden in 10 ml Dichlormethan vorgelegt und bei 4°C 1,01 g N,N-Dicylohexylcarbodiimid zugegeben. Man ließ auf Raumtemperatur erwärmen, trennte vom gebildeten Feststoff ab und chromatographierte das in der Phase enthaltene Rohprodukt an 80 g Kieselgel (Dichlorhexan + Hexan = 3 + 1; 1 % Eisessig). Es wurden 2,0 g N-(2-Hexyl-3-oxo-decanoyl)-phenylalanin-N-hydroxysuccinimidester erhalten.

### Beispiel 15

### Herstellung von Octyldiketen-Capronsäure-Hirudin

1,0 ml einer Hirudin-Lösung (21 mg/ml in 0,1 M Na-Carbonat oder Na-Borat pH 9) wurde mit 1,0 ml Tetrahydrofuran verdünnt und mit 7 mg N-(6-(2-Hexyl-3-oxo-decanoylamino)-hexanoyloxy)-succinimid versetzt und 12 h bei Raumtemperatur unter Rühren inkubiert. Die Reaktion wurde durch Zusatz von Ethanolamin (2fach molarer Überschuß bezogen auf aktiviertes Diketen) abgestoppt und durch RP-Chromatographie - wie in Beispiel 10 beschrieben - aufgetrennt. Die Hirudin-Diketenderivate eluierten bei 48 % B (Derivat 1), 52 % B (Derivat 2) und 63 % B (Derivat 3). Die spezifischen Aktivitäten wurden wie in Beispiel 2 ermittelt und betrugen für Derivat 1 7500 U/mg, für Derivat 2 3400 U/mg und 80 U/mg für Derivat 3.

Das Ausgangsmaterial wurde wie folgt hergestellt:

6,55 g 6-Aminocapronsäure wurden bei pH 9 in 100 ml Wasser gelöst und im Verlauf von 20 min 14,24 g Octyldiketen zugetropft. Es wurde über Nacht gerührt, mit konzentrierter Salzsäure der pH-Wert auf 1 eingestellt und die wäßrige Phase mit 100 ml MTBE extrahiert. Die organische Phase wurde über Magnesiumsulfat getrocknet, das Lösungsmittel im Vakuum entfernt und das Rohprodukt in 150 ml Pentan aufgerührt. Der resultierende Feststoff wurde abfiltriert und bei 60°C im Vakuum getrocknet. Es wurden 5,9 g 6-(2-Hexyl-3-oxo-decanoylamino)-hexansäure erhalten.

1,53 g des so erhaltenen Produkts und 0,55 g N-Hydroxysuccinimid wurden in 10 ml Dichlormethan bei 4°C vorgelegt und mit 1,01 g N,N-Dicyclohexylcarbodiimid versetzt. Man ließ auf Raumtemperatur erwärmen, rührte über Nacht und filtrierte den Feststoff ab. Das in der organischen Phase enthaltende Rohprodukt wurde über 80 g Kieselgel chromatographiert (Essigester + Hexan = 3 + 5; 1 % Eisessig). Man erhielt 1,7 g N-[6-(2-hexyl-3-oxo-decanoylamino)hexanoyloxy]-succinimid.

### Beispiel 16

### Kinetik der anti-Faktor IIa Aktivität in Plasmaproben von Hund und Ratte

Methode: Hirudin-Konjugate oder Placebo wurden narkotisierten Ratten oder wachen Hunden intravenös appliziert. Zu definierten Zeiten wurden den Tieren venöse Blutproben zur Gewinnung von Citratplasma entnommen. Die freie anti-Faktor IIa Aktivität der Hirudin- Konjugate in Plasma wurde mittels chromogenem Assay anhand einer Standardkurve mit rekombinantem Hirudin bestimmt.

### Testprinzip:

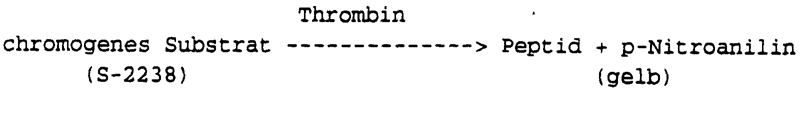

In Mikroplatten werden zu jeweils 100 µl Tris-Puffer (Tris 200 mmol/l, NaCl 25 mmol/1, pH 8,1) je 10 µl Plasmaprobe und 100 µl Thrombin (0,3 NIH-Units/ml) pipettiert. Nach 1 min wird die Reaktion durch Zugabe von 50 µl Substratlösung (S-2238, 1,34 mmol/1) gestartet. Der Ansatz wird nach kurzem Mischen bei 25°C inkubiert und die Reaktion nach 5 min durch Zugabe von 100 µl 30 %iger Essigsäure gestoppt. Die Extinktion der Probe, gemessen bei 405 nm gegen 630 nm, ist umgekehrt proportional der anti-Faktor IIa Aktivität in der Plasmaprobe. In den Plasmaproben wurden teilweise auch die Gerinnungsparameter Thrombinzeit (TT) und partielle Thromboplastinzeit (APTT) gemessen.

### Ergebnisse:

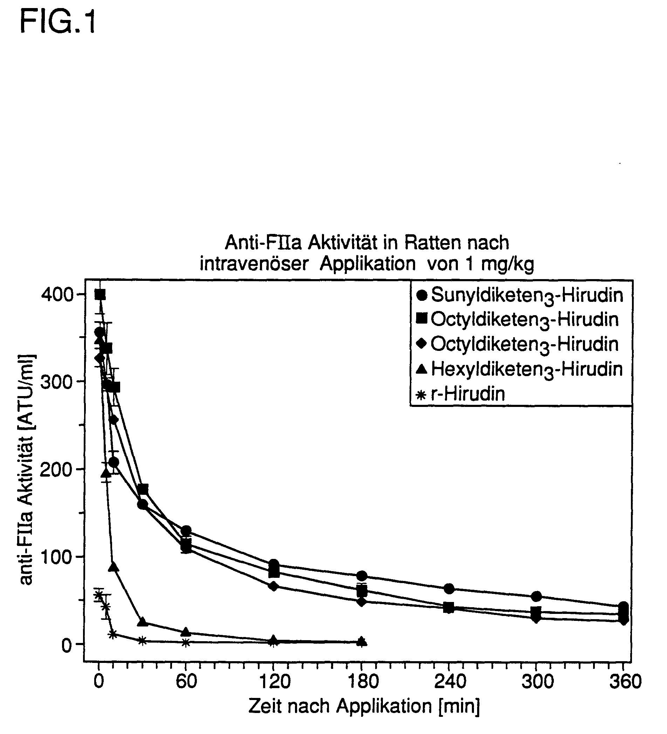

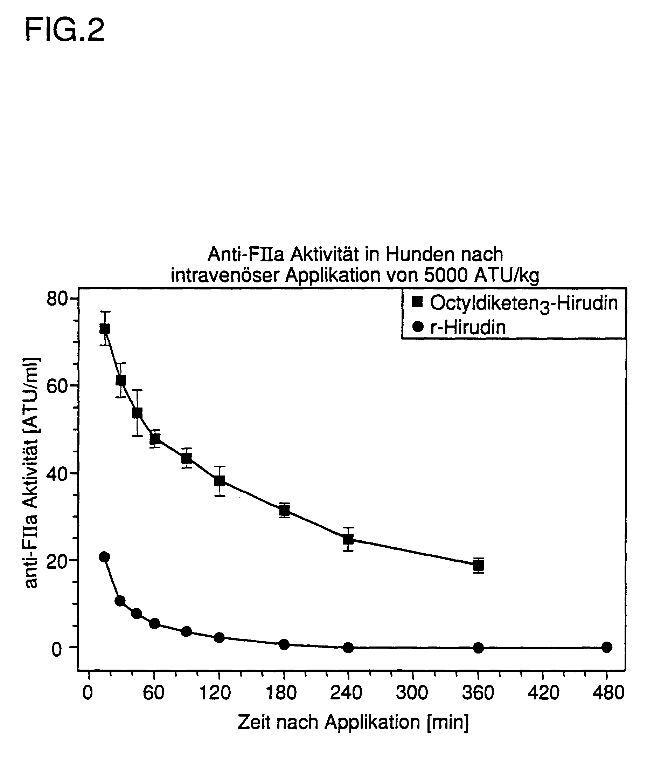

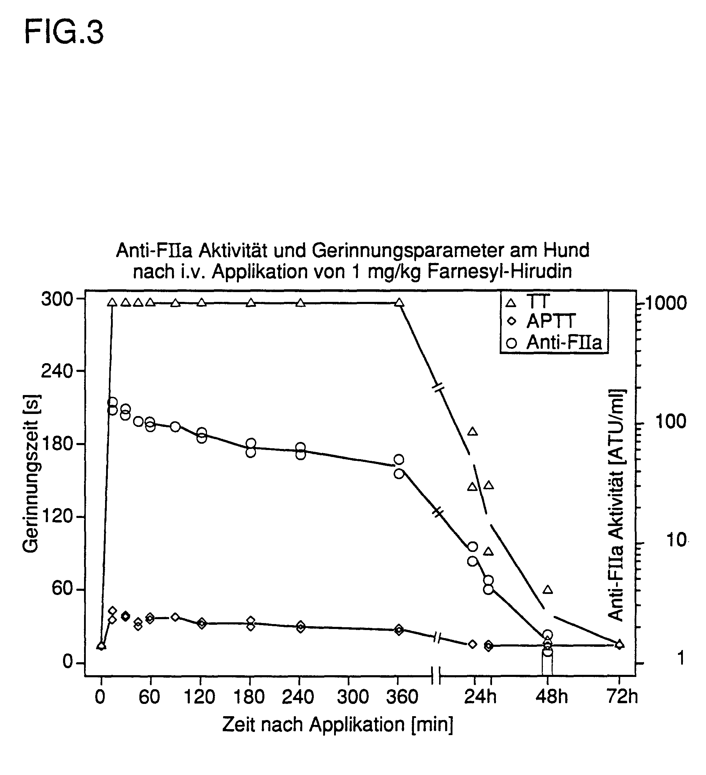

Bei ungefähr gleicher spezifischer Aktivität ist nach i.v. Applikation von 1 mg/kg lipophiler Hirudin-Derivate an narkotisierten Ratten gegenüber r-Hirudin ein deutlich verlangsamter Rückgang der freien anti-Faktor IIa Aktivität festzustellen (Abbildung 1). Bei Hunden wurde nach i.v. Applikation von 5000 Antithrombin-Units/kg (ATU/kg) Octyldiketen₃-Hirudin ebenfalls ein gegenüber r-Hirudin deutlich erhöhter Wirkspiegel mit verlangsamter Elimination gemessen (Abbildung 2). Eine sehr langsame Abnahme der freien anti-Faktor IIa Aktivität wurde auch nach i.v. Applikation von 1 mg/kg Farnesyl-Hirudin (Abbildung 3) beobachtet.

### Beispiel 17

Thrombininduzierte Aggregation von gewaschenen Thrombozyten aus dem Blut von Mensch und Ratte ex vivo und in vitro nach Vorinkubation von Thrombozyten mit lipophilen Hirudin-Derivaten

Methode: Frisches Citratblut (9 Teile Blut + 1 Teil Natriumcitrat 0,11 mol/l, Ratte: 8,5+1,5) wird zur Gewinnung von plättchenreichem Plasma (PRP, Überstand) 16 min bei 250 xg zentrifugiert. Aus dem PRP wird nach einer Methode von Patscheke et al. (Haemostasis 10, 14-27, 1981) ein Konzentrat gewaschener Thrombozyten gewonnen. Dabei werden die Thrombozyten zunächst aus dem PRP durch 7 min Zentrifugation bei 330 x g sedimentiert. Soll eine Behandlung mit Hirudin-Derivaten erfolgen, so wird an dieser Stelle nach Zugabe von 200 µl des Derivats in einer Waschlösung (120 mmol/l NaCl, 5 mmol/1 KCl, 2 mmol/l CaCl₂, 1 mmol/l MgCl₂, 5 mmol/l Glucose, 2 g/l Albumin, 50 mg/l Apyrase in 30 mmol/l Natriumphosphat, pH 6,5) für eine Endkonzentration des Derivats von 0,215 mg/l das Pellet aufgewirbelt und 10 min bei Raumtemperatur inkubiert. Es folgen zwei Waschschritte in dieser Waschlösung und danach die endgültige Resuspension der Thrombozyten in ein Testmedium mit 120 mmol/1 NaCl, 5 mmol/l KCl, 1 mmol/l CaCl₂, 0,1 mmol/l MgCl₂, 5 mmol/l Glucose, 0,5 g/l Albumin, 50 mg/l Apyrase, 1 mmol/l Natriumphosphat, TES/NaOH, pH 7,4. Die Thrombozytenzahl wurde auf 2 x 10⁵/µl (Ratte 8 x 10⁶/µl) eingestellt. Zur Bestimmung der Thrombozytenaggregation werden 218 µl des Thrombozytenkonzentrats 3 min bei 37°C in einem Aggregometer (PAP-4 Bio Data Corporation) inkubiert, 1 min bei 1000 RPM gerührt und nochmals 1 min inkubiert. Dann wird die Aggregation durch Zugabe von 2 µl Thrombinlösung gestartet. Die Thrombozytenaggregation wird über die Änderung der gemessenen Transmission pro Zeiteinheit in der Probe bestimmt (Slope-Methode). Als Maß der relativen Wirksamkeit und Affinität der Hirudin-Derivate wird als EC50 die Thrombinkonzentration in NIH-Units/ml bestimmt, mit der die halbmaximale Steigung der Thrombozytenaggregation erreicht wird. Aus dieser EC50 und der EC50 einer ohne Hirudin vorinkubierten Probe wird der Neutralisationsindex (NI) mit NI = EC50 Derivat / EC50 Kontrolle berechnet. Dieser Wert ist derivatspezifisch und gibt den Faktor an, um den die Thrombinkonzentration als Agonist erhöht werden muß, um wieder die gleiche Aggregation (halbmaximale Steigung) wie in der Kontrolle zu erreichen.

### Ergebnisse:

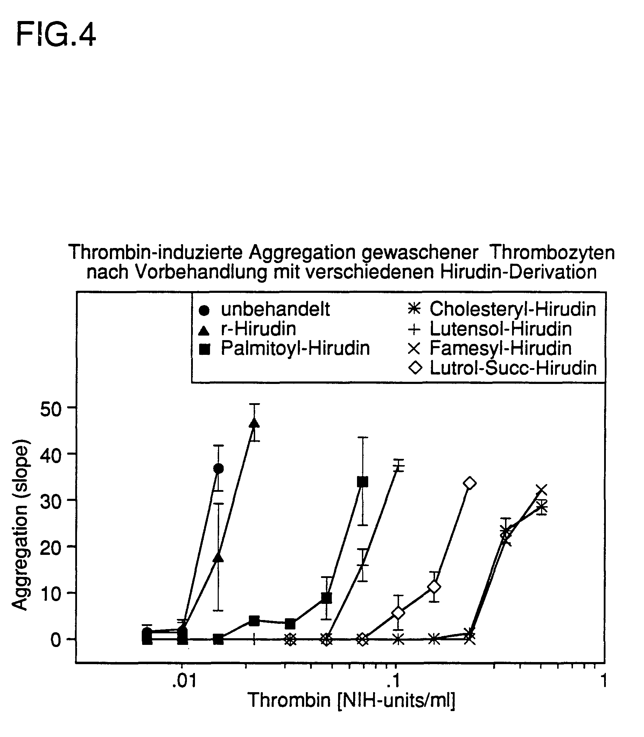

Nach Vorinkubation von Thrombozyten unterschiedlicher Spezies mit 21,5 µg/ml lipophilen Hirudin-Derivaten mußte z.T. ein Vielfaches der in der Kontrolle eingesetzten Thrombinkonzentration verwendet werden, um wieder eine Aggregation zu erzielen (Abbildung 4). Diese Hemmung wird durch immunologisch nachweisbare Anhaftung der lipophilen Hirudin-Derivate an die Thrombozyten bewirkt. Die Neutralisationsindizes erreichen Werte von bis zu 25 bei humanen Thrombozyten (Tabelle 1). Bei Ratten war 24h nach Applikation von 10 mg/kg Cholesteryl-Hirudin noch ein NI von 5,4 an gewaschenen Thrombozyten ex vivo festzustellen (siehe Beispiel 20: Tabelle 2).

**Tabelle 1:**

| Neutralisations-Indizes (NI) für ausgewählte Verbindungen an menschlichen Thrombozyten | |
|---|---|
| Substanz | NI |
| Kontrolle (placebobehandelt) | 1 |
| r-Hirudin | 1,21 |
| Stearoyl-Hirudin | 5,42 |
| Palmitoyl-Hirudin | 4,71 |
| Cholesteryl-Hirudin | 21,2 |
| Lutensol T03-Hirudin | 15,1 |
| Farnesyl-Hirudin | 24,3 |

### Beispiel 18

### Antithrombotische Wirkung am arteriovenösen Shunt an der Ratte

Methode: In diesem Experiment dient eine Glaskapillare in einem arteriovenösen Shunt als künstliche thrombogene Oberfläche und löst eine Thrombose aus. Die narkotisierte (Urethan 25 %, 2 x 8 mg/kg i.p.) Ratte wird in Rückenlage auf einer temperierten (37°C) Wärmebank fixiert. In die freipräparierte rechte A. carotis und V. jugularis werden kurze Polyethylene-Katheter (Portex, PE 50) implantiert, mit physiol. NaCl-Lösung gefüllt und durch Klemmen verschlossen. Die freien Enden der Katheter werden durch eine 20.0 mm lange Glaskapillare (Innendurchmesser 1.0 mm) verbunden, die als thrombogene Oberfläche wirkt.Die Applikation der Prüfsubstanz kann i.v., s.c., p.o. oder als Infusion erfolgen. Nach der gewünschten Inkubationszeit (5, 60, oder 360 min) mit der Prüfsubstanz oder Lösungsmittel (Kontrolle) wird der Shunt durch Entfernen der Klemmen geöffnet. Der Blutstrom durch den Shunt führt zu einen schnellen Anstieg der Shunttemperatur, die an der Mitte der Glaskapillare gemessen wird. Die Erhöhung von Raumtemperatur auf Körpertemperatur ist ein Indikator für die Durchgängigkeit des Shunts. Die Temperatur wird bis zum Verschluß des Shunts längstens jedoch 30 Minuten kontinuierlich aufgezeichnet. Für den Vergleich der Wirksamkeit unterschiedlicher Hirudin-Derivate wird die ED15min als die Dosis berechnet, die bezogen auf die Kontrollgruppe zu einer Verlängerung der Verschlußzeit um 15 Minuten führt. Bei Öffnung des Shunts und am Ende des Experiments werden zusätzlich Blutproben zur Bestimmung der anti-FIIa-Aktivität im Plasma entnommen.

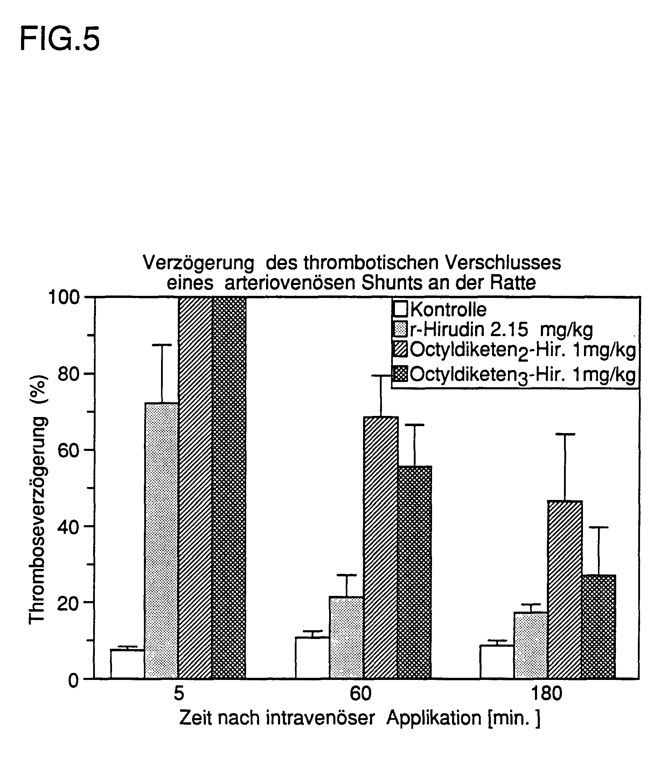

Ergebnisse: Octyldiketen₃-Hirudin und Octyldiketen₃-Hirudin zeigen aufgrund ihrer längeren Wirkdauer antithrombotische Wirksamkeit schon in geringeren Dosen und über einen längeren Zeitraum als r-Hirudin (Abbildung 5).

### Beispiel 19

### Antithrombotische Wirkung auf die strominduzierte Thrombose an der Ratte

Methode: In diesem Experiment wird durch kurzen Stromfluß durch zwei an die A. Carotis angelegte Elektroden eine Endothelschädigung erzeugt und dadurch ein thrombotischer Verschluß des Gefäßes ausgelöst. Die narkotisierte (Urethan 25 %, 2 x 8 mg/kg i.p.) Ratte wird in Rückenlage auf einer temperierten (37°C) Wärmebank fixiert. Ein 20 mm langes Segment der rechten A. carotis wird freipräpariert und ein Ultraschall Transit-time Flußmeßkopf auf der proximalen Häfte des Segmentes plaziert. Der Volumenfluß wird kontinuierlich während der Beobachtungszeit von 30 min mittels eines Ultraschall Flußmeßgerätes (T206, Transonic Systems Inc., USA) aufgenommen. Die Thrombusbildung wird ausgelöst durch einen konstanten Stromfluß (3 mA, 1 min) durch ein hakenförmiges Elektrodenpaar, das 1 cm distal des Flußmeßkopfes auf die Gefäßoberfläche aufgebracht wird. Gefäßokklusion wird definiert als eine Verminderung des Volumenflusses auf < 0,3 ml/min für mehr als drei Minuten. Die antithrombotische Wirksamkeit von Hirudin-Derivaten wird quantifiziert als Thrombosehäufigkeit innerhalb 30 min in einer Gruppe von 10 Tieren. Für den Vergleich der Wirksamkeit unterschiedlicher Hirudin-Derivate wird die ED50 als die Dosis berechnet, die bezogen auf die Kontrollgruppe die Thrombosehäufigkeit um 50 % reduziert. Vor Anlegen der Spannung und am Ende des Experiments werden zusätzlich Blutproben zur Bestimmung der anti-FIIa-Aktivität im Plasma entnommen.

### Ergebnisse:

Für Palmitoyl₂-Hirudin wurde eine ED50 von 0,24 mg/kg bestimmt, gemessen 5 Minuten nach intravenöser Applikation. Die entsprechende ED50 von r-Hirudin beträgt 0,47 mg/kg. Auch 24 h nach intravenöser Applikation von Cholesteryl-Hirudin konnte an Ratten aufgrund der langen Wirkdauer noch Hemmung der Aggregation gewaschener Thrombozyten ex vivo und gute antithrombotische Wirksamkeit gezeigt werden (Tabelle 2).

**Tabelle 2:**

| Antithrombotischer Effekt und Thrombozytenaggregationshemmung von Cholesteryl-Hirudin 24h nach i.v. Applikation (n=9-10) | | | | | |
|---|---|---|---|---|---|
| Behandlung | Thrombose Häufigkeit (%) | Neutralisations Index (NI) in gewaschenen Thrombozyten | Plasma anti-FIIa Aktivität [ATU/ml] | TT [s] | APTT [s] |
| in vitro | - | 18.3 ± 6.1 | - | - | - |
| Placebo | 100 | 1 | 0 | 41 ± 1 | 29 ± 4 |
| 1.0 mg/kg | 50 | 1.5 ± 0.02 | 5.7 ± 0.6 | 267 ± 15 | 30 ± 3 |
| 10 mg/kg | 11 | 5.39 ± 0.74 | 61.0 ± 3.8 | > 300 | 52 ± 4 |

## Patentansprüche

1. Hirudin-Konjugate, gebildet aus einem Hirudin und einem oder mehreren lipophilen Verbindungen, wobei die lipophile Verbindung einen Octanol-Wasser-Verteilungskoeffizienten von mehr als 1,8 aufweist und chemisch mit dem Hirudin kovalent verknüpft ist.

2. Hirudin-Konjugate nach Anspruch 1, **dadurch gekennzeichnet**, daß die Verknüpfung der lipophilen Verbindung im Bereich der Aminosäuren 27-37 erfolgt.

3. Hirudin-Konjugate nach Anspruch 1 und 2, **dadurch gekennzeichnet**, daß die Verknüpfung zwischen Hirudin und lipophilen Verbindungen über die Aminoseitenketten von Lysinresten des Hirudins erfolgt.

4. Hirudin-Konjugate nach Anspruch 1 bis 3, **dadurch gekennzeichnet**, daß 1 bis 3 lipophile Verbindungen mit dem Hirudin verknüpft sind.

5. Hirudin-Konjugate nach Anspruch 1 bis 4, **dadurch gekennzeichnet**, daß mindestens eine lipophile Verbindung mit der Aminosäure 27 oder 33 verknüpft ist.

6. Hirudin-Konjugate gemäß Anspruch 1 bis 5 zur Verwendung bei der Bekämpfung von Krankheiten.

7. Verfahren zur Herstellung von Hirudin-Konjugaten, **dadurch gekennzeichnet**, daß ein Hirudin mit einem oder mehreren Moläquivalenten einer lipophilen Verbindung, gegebenenfalls nach chemischer Aktivierung der lipophilen Verbindung, umgesetzt wird.

## Claims

1. A hirudin conjugate formed from a hirudin and one or more lipophilic compounds, where the lipophilic compound has an octanol/water partition coefficient of more than 1.8 and is covalently linked to the hirudin.

2. A hirudin conjugate as claimed in claim 1, wherein the linkage of the lipophilic compound takes place in the region of amino acids 27-37.

3. A hirudin conjugate as claimed in claim 1 or 2, wherein the linkage between hirudin and lipophilic compounds takes place via the amino side chains of lysine residues in the hirudin.

4. A hirudin conjugate as claimed in any of claims 1 to 3, wherein 1-3 lipophilic compounds are linked to the hirudin.

5. A hirudin conjugate as claimed in any of claims 1 to 4, wherein at least one lipophilic compound is linked to amino acid 27 or 33.

6. A hirudin conjugate as claimed in any of claims 1 to 5 for use for controlling diseases.

7. A process for preparing hirudin conjugates, which comprises reacting a hirudin with one or more mole equivalents of a lipophilic compound, where appropriate after chemical activation of the lipophilic compound.

## Revendications

1. Conjugué d'hirudine, formé d'une hirudine et d'un ou plusieurs composés lipophiles, tandis que le composé lipophile présente un coefficient de répartition octanol-eau supérieur à 1,8 et est lié chimiquement par covalence avec l'hirudine.

2. Conjugué d'hirudine selon la revendication 1, caractérisé par le fait que la liaison du composé lipophile s'effectue dans la région des acides aminés 27-37.

3. Conjugué d'hirudine selon la revendication 1 et 2, caractérisé par le fait que la liaison entre l'hirudine et les composés lipophiles s'effectue par l'intermédiaire des chaînes latérales amino des restes lysine de l'hirudine.

4. Conjugué d'hirudine selon la revendication 1 à 3, caractérisé par le fait que 1 à 3 composés lipophiles sont liés avec l'hirudine.

5. Conjugué d'hirudine selon la revendication 1 à 4, caractérisé par le fait qu'au moins un composé lipophile est lié avec l'acide aminé 27 ou 33.

6. Conjugué d'hirudine selon la revendication 1 à 5 pour utilisation dans la lutte contre des maladies.

7. Procédé pour la préparation de conjugués d'hirudine, caractérisé par le fait qu'on fait réagir une hirudine avec un ou plusieurs équivalents molaires d'un composé lipophile, éventuellement après activation chimique du composé lipophile.
